# EUROPEAN PATENT APPLICATION

(11) **EP 2 301 377 A2**
(43) Date of publication of application: **30.03.2011**
(21) Application number: 09746766.6
(22) Date of filing: 14.05.2009
(51) Int. Cl.: A45D 34/04, A45D 34/00

(54) **LIQUID CONTAINER WITH APPLICATION FUNCTION**

(30) Priority: 14.05.2008 KR 20080044582; 05.01.2009 KR 20090000369
(71) Applicant: Lee, Choong-Bae, Gyeonggi-Do 437-738 (KR); Kim, Soon-Urn, Gyeonggi-Do 463-797 (KR)
(72) Inventor: Lee, Choong-Bae, Gyeonggi-Do 437-738 (KR); Kim, Soon-Urn, Gyeonggi-Do 463-797 (KR)
(74) Representative: Verscht, Thomas Kurt Albert
(86) International application number: PCT/KR2009/002563
(87) International publication number: WO 2009/139587

(57) **Abstract**

The present invention relates to a liquid container with an application function, and more particularly, to a liquid container with an application function which enables a user to just open the cap of the liquid container and directly apply the contents squeezed from the liquid container directly onto the skin of the user without the need for scrubbing the contents with the hands of the user or scrubbing the contents by using a separate application tool after squeezing the contents onto the body parts or hands of the user or the application tool. The liquid container of the present invention has an application member integrally mounted to an outlet portion of a main body of the liquid container such that the liquid contents squeezed from the main body is absorbed and distributed uniformly over the entirety of the application member made of an absorbent material and scrubbed, by the user, directly onto the body parts of the user, thereby allowing the user to apply the liquid content to his or her skin in a convenient and sanitary manner.

## Description

### Technical Field

The present invention relates, in general, to a liquid container with an application function and, more particularly, to a liquid container with an application function, which enables a user to just open a cap of the liquid container and directly apply liquid contents squeezed from the liquid container onto a desired portion of the skin, without the necessity of squeezing the contents onto his or her body part, hand, or a separate application tool and rubbing the contents with the hand or the application tool.

### Background Art

Generally, various kinds of cosmetics are available on the market. Recently, for the convenience of users, cosmetics are changing from solid cosmetics into liquid cosmetics. Furthermore, in order to allow a user to conveniently store and use the liquid cosmetics, various types of cosmetic containers are being developed and used.

Further, various containers for containing cosmetics, such as creams or lotions, which are applied to a user's body part, medicines such as ointments, and various kinds of liquid or liquid materials having viscosity, are being developed and used.

A container containing a liquid product having viscosity generally uses a tube-type container which is made of soft synthetic resin or a pump-type container having a pressurization pump. Such a container is used by squeezing a container body or spraying contents, because the liquid contents having high viscosity are not smoothly discharged out of the container although the container is stood upside down or shaken.

However, the conventional tube-type or pump-type container is problematic in that, when a user desires to use contents contained in the container, he or she must discharge the contents through an outlet portion of the container and then apply the contents onto his or her body part using the hand, so that it is inconvenient to wash off the contents remaining on the hand after the contents have been applied to the body part.

Especially in summer, sun-cream is frequently applied to the face. When the sun-cream that is applied is in liquid form, the sun-cream must be discharged out of the container and then applied using the hand. However, since the sun-cream that is put on the hand is sticky, the hand must be cleaned using tissue paper or water, thus inconveniencing a user.

In order to solve such problems, a rotary roller is provided in the outlet portion of the container, thus allowing contents to be applied to a user's body part without having to use the hand. However, in this case, there is no device for opening or closing the outlet portion, so that the contents may leak out through the outlet portion of the container as a result of a user's carelessness even when a product is not in use, or may be contaminated by the external air which may flow into the container through the open outlet portion.

### Disclosure

### Technical Problem

Accordingly, the present invention has been made keeping in mind the above problems occurring in the prior art, and an object of the present invention is to provide a liquid container with an application function, which has an application member between an outlet portion of a main body and a cap, thus allowing a user to apply liquid contents to a body part in a convenient and sanitary manner while squeezing the liquid contents out of the main body.

Another object of the present invention is to provide a liquid container with an application function, which is constructed so that the interior of an outlet portion is isolated as soon as a main body is covered with a cap when the container is not in use, thus preventing external air from entering the container, and preventing contents from leaking out as a result of a user's carelessness.

### Technical Solution

In order to accomplish the above objects, the present invention provides a liquid container with an application function, including a main body containing contents therein, and having an outlet portion; an inner stopper having a shape of a cylinder that has a through hole along a central axis thereof, inserted into the outlet portion, and having a discharge plate which is formed to correspond to a sectional area of the through hole and is inserted into the through hole to close the through hole, at least one discharge perforation being formed through the discharge plate in a vertical direction; an opening control unit, a lower portion of which is inserted into an upper portion of the inner stopper such that a lower end of the opening control unit is placed on the discharge plate, the opening control unit including a passage which is formed through a central axis thereof, and a support step which protrudes from a middle portion of an outer circumference thereof and is formed at a position spaced apart from upper ends of the outlet portion and the inner stopper by a predetermined interval so as to move up and down by the interval; an elastic member disposed between the upper ends of the outlet portion and the inner stopper and the support step, and providing upward moving force to the opening control unit; an outer stopper having a shape of a cylinder which is open at a bottom thereof, and including a hole formed in a central portion of an upper surface of the outer stopper to correspond to the outer circumference of the opening control unit, and a coupling inner wall provided in an inner receiving space in such a way as to have an inner circumference corresponding to an outer circumference of the outlet portion and to extend from the upper surface of the outer stopper; a support member having a shape of a plate through which a coupling hole is formed such that the opening control unit is inserted into the coupling hole, with a guide wall protruding along an edge of the plate; an application member fitted into the guide wall, and made of an absorbent material, an upper portion of the opening control unit being inserted into a through hole which is formed along a central axis of the application member; and a cap being open at a bottom thereof to cover the support member and the application member, and detachably coupled to an upper portion of the outer stopper.

Further, an inner circumference of a lower portion of the cap may engage with an outer circumference of the outer stopper, either of the inner circumference of the lower portion of the cap or the outer circumference of the outer stopper may include an outer undercut which protrudes in relief, and a remaining one may include an inner undercut which is formed in intaglio to correspond to the outer undercut.

Further, the present invention provides a liquid container with an application function, including a main body containing contents therein, and having an outlet portion; an inner stopper having a shape of a cylinder that has a through hole along a central axis thereof, inserted into the outlet portion, and having a discharge plate which is formed to correspond to a sectional area of the through hole and is inserted into the through hole to close the through hole, at least one discharge perforation being formed through the discharge plate in a vertical direction; an opening control unit, a lower portion of which is inserted into an upper portion of the inner stopper such that a lower end of the opening control unit is placed on the discharge plate, the opening control unit including a passage which is formed therein, and a support step which protrudes from a middle portion of an outer circumference thereof and is formed at a position spaced apart from upper ends of the outlet portion and the inner stopper by a predetermined interval so as to move up and down by the interval; an elastic member disposed between the upper ends of the outlet portion and the inner stopper and the support step, and providing upward moving force to the opening control unit; an outer stopper having a shape of a cylinder which is open at a bottom thereof, and including a hole formed in a central portion of an upper surface of the outer stopper to correspond to the outer circumference of the opening control unit, a coupling inner wall provided in an inner receiving space in such a way as to have an inner circumference corresponding to an outer circumference of the outlet portion and extend from the upper surface of the outer stopper, and a locking protrusion provided at a predetermined height on an outer circumference of the outer stopper in such a way as to protrude along the outer circumference; an application member shaped such that the application member is placed on an upper surface of the outer stopper and an upper portion of the opening control unit is inserted into a through hole which is formed along a central axis thereof, and including an insert groove which is formed along an outer circumference thereof in intaglio, and an "O"-shaped coupling ring which is inserted into the insert groove in such a way that a portion of the coupling ring protrudes outwards, the application member being made of an absorbent material; a coupling member having a shape of a cylinder which surrounds outer circumferences of an upper portion of the outer stopper and a lower portion of the application member, and including upper and lower hooks which protrude inwards to correspond to the locking protrusion and the coupling ring, respectively, the coupling member coupling the outer stopper with the application member; and a cap receiving the application member and the coupling member therein, detachably closing the upper portion of the outer stopper, and having a central push part which is inserted into the through hole of the application member to exert pressure on an upper end of the opening control unit located in the through hole.

Further, an inner circumference of a lower portion of the cap may engage with an outer circumference of the coupling member which is coupled to the outer circumference of the upper portion of the outer stopper, either of the inner circumference of the lower portion of the cap or the outer circumference of the coupling member may include an outer undercut which protrudes in relief, and a remaining one may include an inner undercut which is formed in intaglio to correspond to the outer undercut.

The liquid container may further include a push part provided on a lower end of the cap and having an inward step to correspond to an upper end of the guide wall.

The liquid container may further include a locking step provided on an outer circumference of an upper portion of the inner stopper.

The liquid container may further include a sealing protrusion protruding from an upper surface of a central portion of the discharge plate of the inner stopper, and having a circumference which corresponds to an inner circumference of the passage of the opening control unit.

Further, a fastening protrusion may protrude from an outer surface of the outlet portion, and a fastening groove may be formed in an inner surface of the coupling inner wall of the outer stopper to correspond to the fastening protrusion.

Further, the passage of the opening control unit may be formed such that an upper portion thereof is narrower than a lower portion thereof, thus preventing an excessive amount of contents from being discharged out of the main body.

The liquid container may further include an anti-removal protrusion provided on an outer surface of the upper portion of the opening control unit which is fitted into the through hole of the application member.

Further, the absorbent material of the application member may be selected out of a group consisting of latex, sponge, and synthetic resin.

### Advantageous Effects

According to the present invention, a liquid container with an application function is advantageous in that it has an application member integrally mounted to an outlet portion of a main body such that liquid contents squeezed out of the main body are absorbed and distributed uniformly over the entirety of the application member made of an absorbent material and rubbed, by a user, directly onto his or her body part, thereby allowing the user to apply the liquid contents to his or her body part in a convenient and sanitary manner.

Further, a liquid container with an application function is advantageous in that it is constructed so that the interior of an outlet portion is isolated from the surroundings as soon as a main body is covered with a cap when the container is not in use, thus preventing external air from entering the container, thereby preventing the performance of a product from degrading over a lengthy period of time, and preventing the contents from leaking out as a result of a user's carelessness, thereby preventing the surroundings of the outlet portion and the cap from becoming dirty, thereby always keeping the container sanitary.

### Description of Drawings

Fig. 1 is a perspective view showing a liquid container with an application function, according to a first embodiment of the present invention;
Fig. 2 is a view showing the use of the liquid container with the application function, according to the present invention;
Fig. 3 is a view showing the construction and assembling structure of the liquid container, according to the first embodiment of the present invention;
Fig. 4 is a view showing the assembled liquid container, according to the first embodiment of the present invention;
Figs. 5a and 5b are views showing the operation of the liquid container according to the first embodiment of the present invention, when a cap is opened or closed;
Fig. 6 is a view showing the construction and assembling structure of a liquid container, according to a second embodiment of the present invention;
Fig. 7 is a view showing the assembled liquid container, according to the second embodiment of the present invention; and
Figs. 8a and 8b are views showing the operation of the liquid container according to the second embodiment of the present invention, when a cap is opened or closed.

### Mode for Invention

Hereinafter, the present invention will be described with reference to the accompanying drawings. Further, when it is determined that the detailed description of the known art related to the present invention may obscure the gist of the present invention, the detailed description will be omitted.

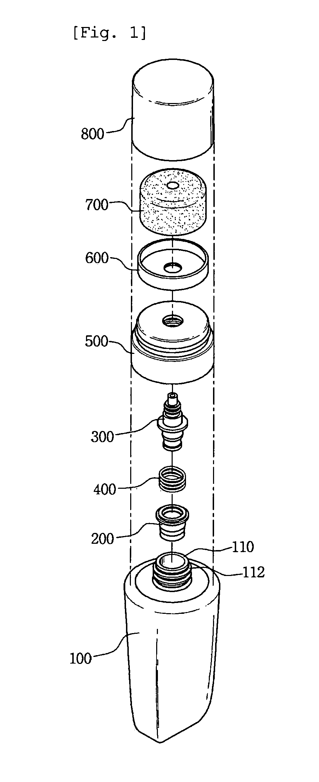

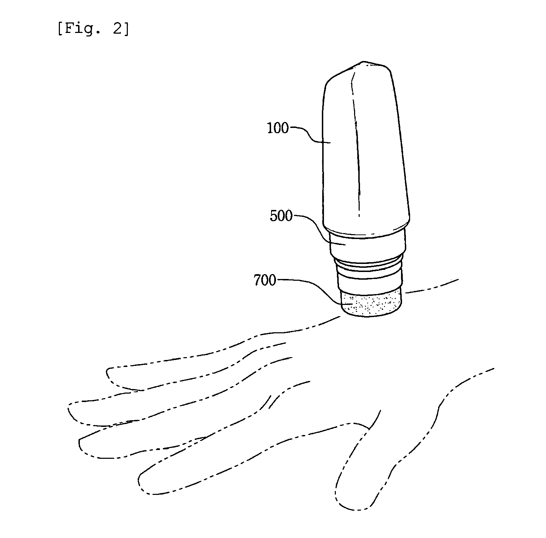

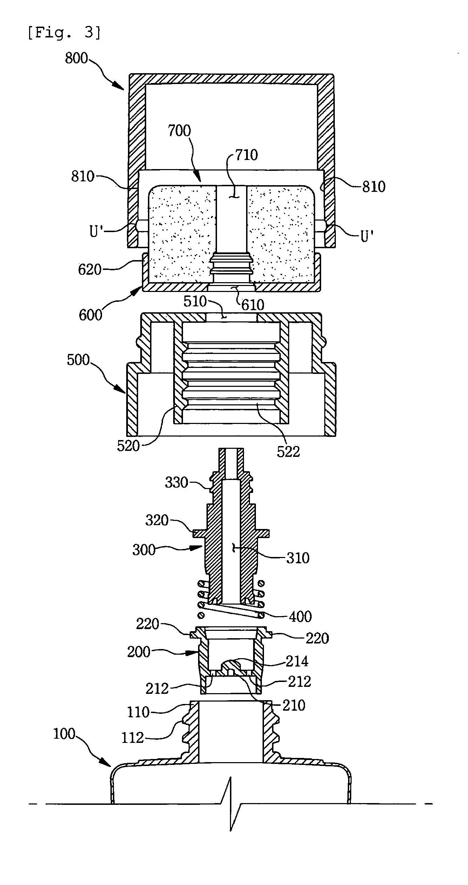

Fig. 1 is a perspective view showing a liquid container with an application function, according to a first embodiment of the present invention, Fig. 2 is a view showing the use of the liquid container with the application function, according to the present invention, and Fig. 3 is a view showing the construction and assembling structure of the liquid container, according to the first embodiment of the present invention.

Referring to Figs. 1, 2 and 3, the liquid container with the application function according to the present invention includes a main body 100, an inner stopper 200, an opening control unit 300, an elastic member 400, an outer stopper 500, a support member 600, an application member 700 and a cap 800, which are assembled with each other.

The liquid container with the application function is operated as follows. If liquid contents are discharged out of the main body 100 by pressing or squeezing the main body 100, the liquid contents are dispersed onto and are distributed over the application member 700, and the upper surface of the application member 700 is put on a body part so as to allow the liquid contents to be uniformly applied to the body part.

The main body 100 contains contents therein, and an outlet portion 110 extends upwards from the main body by a predetermined height. The main body 100 is made of a soft synthetic resin material so that a user can easily squeeze and discharge the contents.

The inner stopper 200 is inserted into the outlet portion 110 to be secured thereto. The outer circumference of the inner stopper 200 corresponds to the inner circumference of the outlet portion 110 so that the outer circumference of the inner stopper 200 is put in close contact with the inner circumference of the outlet portion 110.

The inner stopper 200 has the shape of a cylinder which is hollow therein in a vertical direction. A disc-shaped discharge plate 210, corresponding to a transverse cross-section of the inner stopper, is provided at a predetermined height in the inner stopper 200.

Further, a locking step 220 is provided on the outer circumference of the upper portion of the inner stopper 200 in such a way as to protrude in the form of a ring, thus preventing the inner stopper 200 inserted into the outlet portion 110 from entering the interior of the main body 100.

A plurality of discharge perforations 212 is formed in the discharge plate 210 in such a way as to pass therethrough in the vertical direction.

The opening control unit 300 has a cylindrical shape, and is formed such that a passage 310 is formed therein, its lower portion is inserted into the inner stopper 200, and its central and upper portions are exposed to the outside of the inner stopper 200.

A support step 320 protrudes from the circumference of the middle portion of the opening control unit 300. The support step 320 is inserted to a depth such that the lower end of the opening control unit 300 is in close contact with the discharge plate 210, and is not moved any more from the depth.

The elastic member 400 may comprise a general spring. The elastic member is placed such that its lower end is seated on the upper surface of the locking step 220 of the inner stopper 200 and its upper end is supported by the lower surface of the support step 320 of the opening control unit 300.

The elastic member 400 makes the lower end of the opening control unit 301 be spaced apart from the discharge plate 210 by a predetermined interval.

The outer stopper 500 has the shape of a cylinder which is open at a bottom, and is assembled to receive the outlet portion 110, the inner stopper 200 and the opening control unit 300 in a lower receiving space thereof.

A hole 510 corresponding to the outer circumference of the opening control unit 300 is formed in the central portion of the top of the outer stopper 500 such that the upper portion of the opening control unit 300 passes through the hole and is exposed to the outside of the opening control unit.

The inner stopper 200 and the opening control unit 300 are inserted into the inner receiving space of the outer stopper 500, and a coupling inner wall 520, which has the inner circumference corresponding to the outer circumference of the outlet portion 110, extends downwards from the upper surface of the outer stopper 500 and is secured to the outlet portion 110.

Here, a plurality of annular fastening protrusions 112 is provided on the outer circumference of the outlet portion 110, and fastening grooves 522 are formed in the inner circumference of the coupling inner wall 520 of the outer stopper 500 in such a way as to correspond to the fastening protrusions 112, so that coupling force between the outlet portion and the outer stopper is increased.

When the outer stopper 500 is coupled to the outlet portion, the upper portion of the opening control unit 300 is exposed through the hole 510 to the outside of the outer stopper. Since the hole 510 is formed to correspond to the outer circumference of the opening control unit 300, the support step 320 does not pass through the hole 510.

Thus, the opening control unit 300 is assembled such that it is movable up and down within a predetermined interval and is not dislodged from the outer stopper 500.

The support member 600 has the shape of a plate which is secured to the upper portion of the opening control unit 300, and is formed such that the application member 700 is placed on the upper surface of the support member.

A coupling hole 610 is formed through the central portion of the support member 600, and corresponds to the outer circumference of the upper portion of the opening control unit 300 so that the opening control unit 300 is inserted into the coupling hole. Further, a guide wall 620 is formed along an edge of the upper surface of the support member 600 in such a way as to protrude to a predetermined height, and serves to support the side end of the application member 700.

The application member 700 is placed on the upper surface of the support member 600 to be secured thereto, and has in its central portion a through hole 710 to receive the upper end of the opening control unit 300 therein.

Preferably, the application member 700 is made of a material having absorptive and restoring properties, for example, latex, sponge, and synthetic resin, so that, when liquid contents are discharged from the main body 100 through the outlet portion 110 to the upper end of the opening control unit 300, the liquid contents are absorbed into the through hole 710 of the application member 700 and are uniformly distributed over a surface of the application member.

An anti-removal protrusion 330 is provided on the outer circumference of the upper portion of the opening control unit 300 which is inserted into the through hole 710 of the application member 700, thus preventing the application member 700 from being easily removed from the opening control unit.

The cap 800 is detachably coupled to cover the upper portion of the outer stopper 500 while receiving the support member 600 and the application member 700 therein.

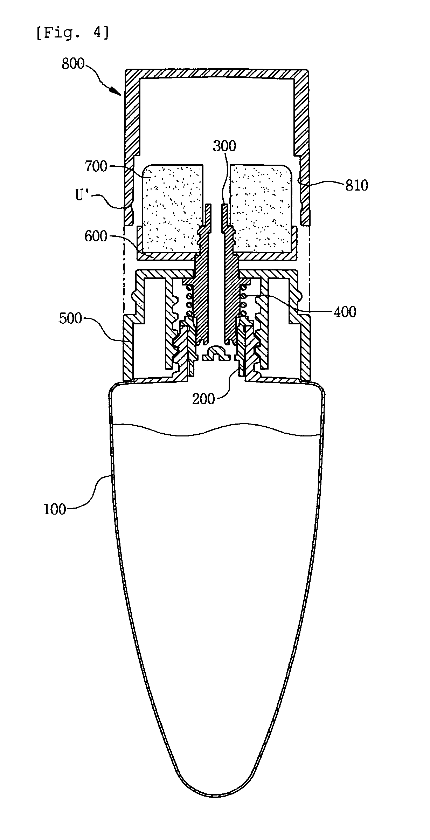

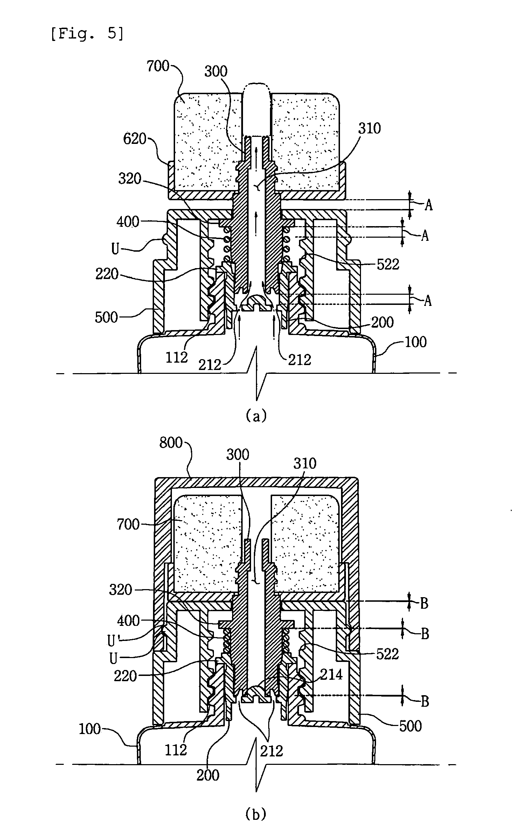

Fig. 4 is a view showing the assembled liquid container, according to the first embodiment of the present invention, and Figs. 5a and 5b are views showing the operation of the liquid container according to the first embodiment of the present invention, when the cap is opened or closed.

Referring to Figs. 4, 5a and 5b, according to the present invention, the inner circumference of the lower portion of the cap 800 is fitted over the outer circumference of the upper portion of the outer stopper 500.

Here, undercuts are formed on the inner circumference of the lower portion of the cap 800 and the outer circumference of the upper portion of the outer stopper 500 to reinforce the coupling force between the cap and the outer stopper. The undercuts comprise a pair of undercuts, including an outer undercut U that protrudes in relief and an inner undercut U' that is formed in intaglio to correspond to the outer undercut.

In the drawings, the outer undercut U is formed on the outer circumference of the upper portion of the outer stopper 500, and the inner undercut U' is formed on the inner circumference of the lower portion of the cap 800. However, the outer undercut U may be formed on the cap 800, and the inner undercut U' may be formed on the outer stopper 500.

The passage 310 is formed through the opening control unit 300 in such a way as to be wide at its lower portion, thus allowing the liquid contents to easily flow from the main body 100 through the inner stopper 200 to the opening control unit. The upper portion of the passage is formed to be narrow so as to prevent an excessive amount of liquid contents from being discharged.

Further, a sealing protrusion 214 is provided on the central portion of the upper surface of the discharge plate 210 of the inner stopper 200 in such a way as to protrude upwards. Preferably, the sealing protrusion 214 protrudes in a hemispherical shape to have a circumference corresponding to the inner circumference of the lower portion of the passage of the opening control unit 300.

Thereby, the discharge perforations 212 are formed outside the sealing protrusion 214 which is formed on the central portion of the discharge plate 210.

A push part 810 is provided on the lower end of the cap 800 to push the upper end of the guide wall 620 of the support member 600 downwards.

The push part 810 is shaped such that the outer and inner surfaces of the lower end of the cap 800 form a step. The lower surface of the push part 810 pushes the guide wall 620 downwards while engaging with the upper end of the guide wall 620.

Figs. 5a and 5b show the operation of the first embodiment according to the present invention constructed as described above.

Fig. 5a shows the state in which the cap 800 is open to use the contents. The lower end of the opening control unit 300 is spaced apart from the discharge plate 210 of the inner stopper 200 by a predetermined interval by the elastic member 400, as shown by A of Fig. 5a.

Thus, liquid contents may flow from the main body 100 through the discharge perforations 212 of the inner stopper 200 into the passage 310 of the opening control unit 300. The liquid contents are discharged to the upper end of the passage 310 of the opening control unit 300 and then are absorbed by the application member 700.

Fig. 5b shows the state in which the cap 800 is closed so that the contents are not used. If the push part 810 of the cap 800 pushes the upper end of the guide wall 620 of the support member 600 downwards, the opening control unit 300 is also moved downwards in conjunction with the support member 600.

At this time, the lower end of the opening control unit 300 comes into close contact with the discharge plate 210 of the inner stopper 200, as shown by B of Fig. 5b.

Therefore, the discharge perforations 212 of the discharge plate 210 are closed by the lower end of the opening control unit 300, and the lower end of the passage 310 of the opening control unit 300 is closed by the sealing protrusion 214, thus preventing liquid contents from being discharged from the inside to the outside of the main body 100.

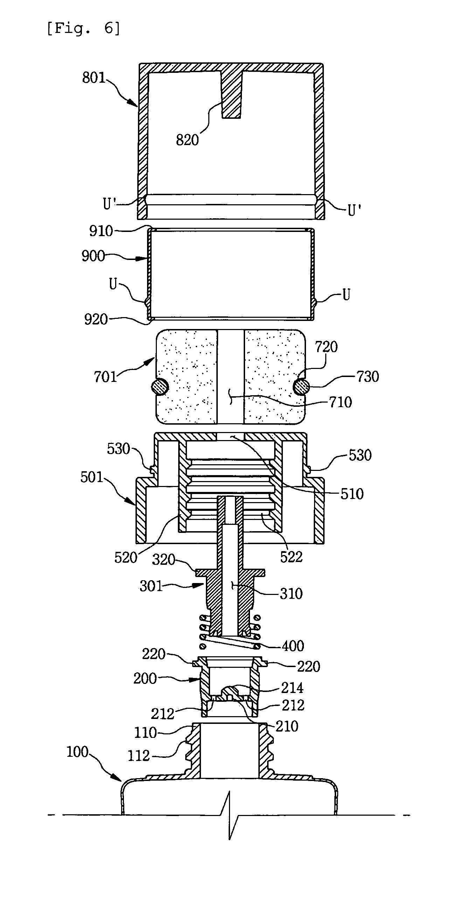

Fig. 6 is a view showing the construction and assembling structure of a liquid container, according to a second embodiment of the present invention.

Referring to Fig. 6, the liquid container with the application function according to the present invention includes a main body 100, an inner stopper 200, an opening control unit 301, an elastic member 400, an outer stopper 501, an application member 701, a coupling member 900 and a cap 801, which are assembled with each other.

The main body 100 contains contents therein, and an outlet portion 110 extends upwards from the main body by a predetermined height. The main body 100 is made of a soft synthetic resin material so that a user can easily squeeze and discharge the contents.

The inner stopper 200 is inserted into the outlet portion 110 to be secured thereto. The outer circumference of the inner stopper 200 corresponds to the inner circumference of the outlet portion 110 so that the outer circumference of the inner stopper 200 is put in close contact with the inner circumference of the outlet portion 110.

The inner stopper 200 has the shape of a cylinder which is hollow therein in a vertical direction. A disc-shaped discharge plate 210, corresponding to a transverse cross-section of the inner stopper, is provided at a predetermined height in the inner stopper 200.

Further, a locking step 220 is provided on the outer circumference of the upper portion of the inner stopper 200 in such a way as to protrude in the form of a ring, thus preventing the inner stopper 200 inserted into the outlet portion 110 from entering the interior of the main body 100.

A plurality of discharge perforations 212 is formed in the discharge plate 210 in such a way as to pass therethrough in the vertical direction.

The opening control unit 301 has a cylindrical shape, and is formed such that a passage 310 is formed therein, its lower portion is inserted into the inner stopper 200, and its central and upper portions are exposed to the outside of the inner stopper 200.

A support step 320 protrudes from the circumference of the middle portion of the opening control unit 301. The support step 320 is inserted to a depth such that the lower end of the opening control unit 301 is in close contact with the discharge plate 210, and is not moved any more from the depth.

The elastic member 400 may comprise a general spring. The elastic member is placed such that its lower end is seated on the upper surface of the locking step 220 of the inner stopper 200 and its upper end is supported by the lower surface of the support step 320 of the opening control unit 301.

The elastic member 400 makes the lower end of the opening control unit 301 be spaced apart from the discharge plate 210 by a predetermined interval A.

The outer stopper 501 has the shape of a cylinder which is open at a bottom, and is assembled to receive the outlet portion 110, the inner stopper 200 and the opening control unit 301 in a lower receiving space thereof.

A hole 510 corresponding to the outer circumference of the opening control unit 301 is formed in the central portion of the top of the outer stopper 501 such that the upper portion of the opening control unit 301 passes through the hole and is exposed to the outside of the opening control unit.

The inner stopper 200 and the opening control unit 301 are inserted into the inner receiving space of the outer stopper 501, and a coupling inner wall 520, which has the inner circumference corresponding to the outer circumference of the outlet portion 110, extends downwards from the upper surface of the outer stopper 501 and is secured to the outlet portion 110.

Here, a plurality of annular fastening protrusions 112 is provided on the outer circumference of the outlet portion 110, and fastening grooves 522 are formed in the inner circumference of the coupling inner wall 520 of the outer stopper 501 in such a way as to correspond to the fastening protrusions 112, so that coupling force between the outlet portion and the outer stopper is increased.

When the outer stopper 501 is coupled to the outlet portion, the upper portion of the opening control unit 301 is exposed through the hole 510 to the outside of the outer stopper. Since the hole 510 is formed to correspond to the outer circumference of the opening control unit 301, the support step 320 does not pass through the hole 510.

Thus, the opening control unit 301 is assembled such that it is movable up and down within a predetermined interval and is not dislodged from the outer stopper 501.

A locking protrusion 530 is provided at a predetermined height on the upper portion of the outer circumference of the outer stopper 501 in such a way as to protrude from the outer circumference thereof.

The application member 701 is placed on the upper surface of the outer stopper 501. The application member and the outer stopper are fastened to each other by the coupling member 900.

The application member 701 having a predetermined height includes in its central portion a through hole 710 to receive the upper end of the opening control unit 301 therein.

Preferably, the application member 701 is made of a material having absorptive and restoring properties, for example, latex, sponge, and synthetic resin, so that, when liquid contents are discharged from the main body 100 through the outlet portion 110 to the upper end of the opening control unit 301, the liquid contents are absorbed into the through hole 710 of the application member 700 and are uniformly distributed over a surface of the application member.

An insert groove 720 is positioned at a predetermined height on the lower end of the application member 701 in such a way as to be formed along the outer circumference thereof in intaglio. An "O"-shaped coupling ring 730 is fitted into the insert groove 720, and is made of an elastic material such as rubber.

Here, only a predetermined portion of the coupling ring 730 is inserted into the insert groove 720 such that a portion of the coupling ring protrudes from the outer surface of the application member 701.

The coupling member 900 has the shape of a cylinder that corresponds to the outer circumferences of the upper portion of the outer stopper 501 and the lower portion of the application member 701.

Preferably, the coupling member 900 is formed to have a minimal height which is sufficient to integrally surround the upper portion of the outer stopper 501 having the locking protrusion 530 and the lower portion of the application member 701 having the coupling ring 730.

An upper hook 910 corresponding to the coupling ring 730 and a lower hook 920 corresponding to the locking protrusion 530 protrude inwards from the inner circumference of the coupling member 900, so that the application member 701 is secured to the upper end of the outer stopper 501.

The cap 801 detachably covers the upper portion of the outer stopper 501 while receiving the application member 701 and the coupling member 900 therein.

A central push part 820 is provided on the central portion of the inner surface of the top of the cap 801 in such a way as to protrude downwards. The central push part 820 is inserted into a through hole 710 of the application member 701 when the cap 801 is closed, thus exerting pressure on the upper end of the opening control unit 301 positioned in the through hole.

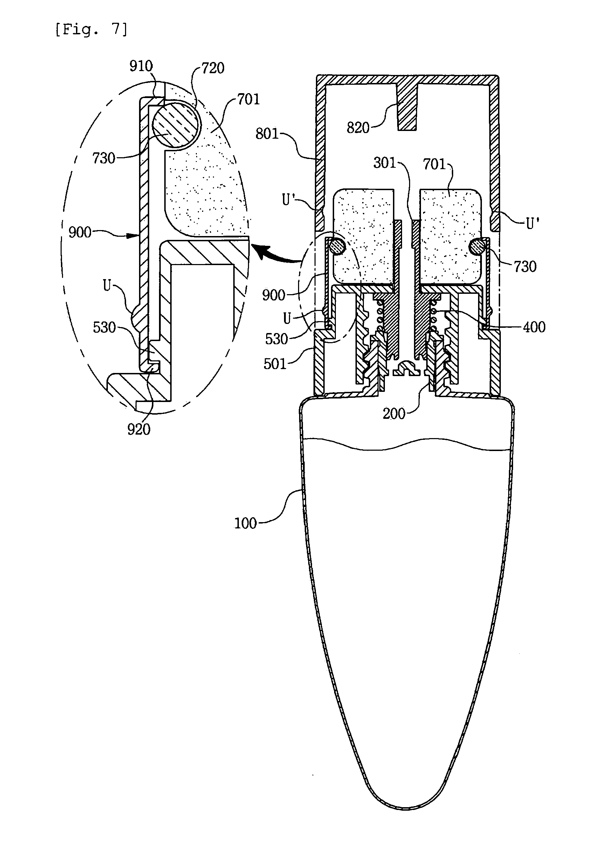

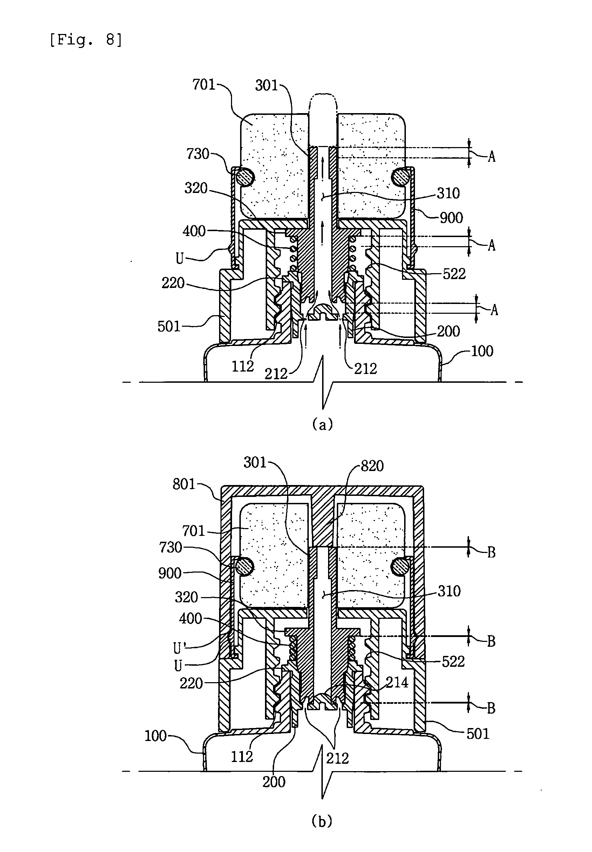

Fig. 7 is a view showing the assembled liquid container, according to the second embodiment of the present invention, and Figs. 8a and 8b are views showing the operation of the liquid container according to the second embodiment of the present invention, when the cap is opened or closed.

Referring to Figs. 7 and 8, according to the present invention, the inner circumference of the lower portion of the cap 801 engages with the outer circumference of the coupling member 900 which is coupled to the outer circumferences of the lower portion of the application member 701 and the upper portion of the outer stopper 501.

Here, undercuts are formed on the inner circumference of the lower portion of the cap 801 and the outer circumference of the upper portion of the coupling member 900 to reinforce the coupling force between the cap and the coupling member. The undercuts comprise a pair of undercuts, including an outer undercut U that protrudes in relief and an inner undercut U' that is formed in intaglio to correspond to the outer undercut.

In the drawings, the outer undercut U is formed on the outer circumference of the upper portion of the coupling member 900, and the inner undercut U' is formed on the inner circumference of the lower portion of the cap 801. However, the outer undercut U may be formed on the cap 801, and the inner undercut U' may be formed on the coupling member 900.

The passage 310 is formed through the opening control unit 301 in such a way as to be wide at its lower portion, thus allowing the liquid contents to easily flow from the main body 100 through the inner stopper 200 to the opening control unit. The upper portion of the passage is formed to be narrow so as to prevent an excessive amount of liquid contents from being discharged.

Further, a sealing protrusion 214 is provided on the central portion of the upper surface of the discharge plate 210 of the inner stopper 200 in such a way as to protrude upwards. Preferably, the sealing protrusion 214 protrudes in a hemispherical shape to have a circumference corresponding to the inner circumference of the lower portion of the passage of the opening control unit 301.

Thereby, the discharge perforations 212 are formed outside the sealing protrusion 214 which is formed on the central portion of the discharge plate 210.

The central push part 820 is provided on the inner surface of the cap 801 to downwardly push the upper end of the opening control unit 301 which is inserted into the through hole 710 of the application member 701.

Figs. 8a and 8b show the operation of the second embodiment according to the present invention constructed as described above.

Fig. 8a shows the state in which the cap 801 is open to use the contents. The lower end of the opening control unit 301 is spaced apart from the discharge plate 210 of the inner stopper 200 by a predetermined interval by the elastic member 400, as shown by A of Fig. 8a.

Thus, liquid contents may flow from the main body 100 through the discharge perforations 212 of the inner stopper 200 into the passage 310 of the opening control unit 301. The liquid contents are discharged to the upper end of the passage 310 of the opening control unit 301 and then are absorbed by the application member 701.

Fig. 8b shows the state in which the cap 801 is closed so that the contents are not used. The central push part 820 of the cap 801 pushes the upper end of the opening control unit 301 downwards.

At this time, the lower end of the opening control unit 301 comes into close contact with the discharge plate 210 of the inner stopper 200, as shown by B of Fig. 8b.

Therefore, the discharge perforations 212 of the discharge plate 210 are closed by the lower end of the opening control unit 301, and the lower end of the passage 310 of the opening control unit 301 is closed by the sealing protrusion 214, thus preventing liquid contents from being discharged from the inside to the outside of the main body 100.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A liquid container with an application function, comprising:
a main body containing contents therein, and having an outlet portion;
an inner stopper having a shape of a cylinder that has a through hole along a central axis thereof, inserted into the outlet portion, and having a discharge plate which is formed to correspond to a sectional area of the through hole and is inserted into the through hole to close the through hole, at least one discharge perforation being formed through the discharge plate in a vertical direction;
an opening control unit, a lower portion of which is inserted into an upper portion of the inner stopper such that a lower end of the opening control unit is placed on the discharge plate, the opening control unit including a passage which is formed through a central axis thereof, and a support step which protrudes from a middle portion of an outer circumference thereof and is formed at a position spaced apart from upper ends of the outlet portion and the inner stopper by a predetermined interval so as to move up and down by the interval;
an elastic member disposed between the upper ends of the outlet portion and the inner stopper and the support step, and providing upward moving force to the opening control unit;
an outer stopper having a shape of a cylinder which is open at a bottom thereof, and including a hole formed in a central portion of an upper surface of the outer stopper to correspond to the outer circumference of the opening control unit, and a coupling inner wall provided in an inner receiving space in such a way as to have an inner circumference corresponding to an outer circumference of the outlet portion and to extend from the upper surface of the outer stopper;
a support member having a shape of a plate through which a coupling hole is formed such that the opening control unit is inserted into the coupling hole, with a guide wall protruding along an edge of the plate;
an application member fitted into the guide wall, and made of an absorbent material, an upper portion of the opening control unit being inserted into a through hole which is formed along a central axis of the application member; and
a cap being open at a bottom thereof to cover the support member and the application member, and detachably coupled to an upper portion of the outer stopper.

2. The liquid container according to claim 1, wherein an inner circumference of a lower portion of the cap engages with an outer circumference of the outer stopper, either of the inner circumference of the lower portion of the cap or the outer circumference of the outer stopper comprises an outer undercut which protrudes in relief, and a remaining one comprises an inner undercut which is formed in intaglio to correspond to the outer undercut.

3. A liquid container with an application function, comprising:
a main body containing contents therein, and having an outlet portion;
an inner stopper having a shape of a cylinder that has a through hole along a central axis thereof, inserted into the outlet portion, and having a discharge plate which is formed to correspond to a sectional area of the through hole and is inserted into the through hole to close the through hole, at least one discharge perforation being formed through the discharge plate in a vertical direction;
an opening control unit, a lower portion of which is inserted into an upper portion of the inner stopper such that a lower end of the opening control unit is placed on the discharge plate, the opening control unit including a passage which is formed therein, and a support step which protrudes from a middle portion of an outer circumference thereof and is formed at a position spaced apart from upper ends of the outlet portion and the inner stopper by a predetermined interval so as to move up and down by the interval;
an elastic member disposed between the upper ends of the outlet portion and the inner stopper and the support step, and providing upward moving force to the opening control unit;
an outer stopper having a shape of a cylinder which is open at a bottom thereof, and including a hole formed in a central portion of an upper surface of the outer stopper to correspond to the outer circumference of the opening control unit, a coupling inner wall provided in an inner receiving space in such a way as to have an inner circumference corresponding to an outer circumference of the outlet portion and extend from the upper surface of the outer stopper, and a locking protrusion provided at a predetermined height on an outer circumference of the outer stopper in such a way as to protrude along the outer circumference;
an application member shaped such that the application member is placed on an upper surface of the outer stopper and an upper portion of the opening control unit is inserted into a through hole which is formed along a central axis thereof, and including an insert groove which is formed along an outer circumference thereof in intaglio, and an "O"-shaped coupling ring which is inserted into the insert groove in such a way that a portion of the coupling ring protrudes outwards, the application member being made of an absorbent material;
a coupling member having a shape of a cylinder which surrounds outer circumferences of an upper portion of the outer stopper and a lower portion of the application member, and including upper and lower hooks which protrude inwards to correspond to the locking protrusion and the coupling ring, respectively, the coupling member coupling the outer stopper with the application member; and
a cap receiving the application member and the coupling member therein, detachably closing the upper portion of the outer stopper, and having a central push part which is inserted into the through hole of the application member to exert pressure on an upper end of the opening control unit located in the through hole.

4. The liquid container according to claim 3, wherein an inner circumference of a lower portion of the cap engages with an outer circumference of the coupling member which is coupled to the outer circumference of the upper portion of the outer stopper, either of the inner circumference of the lower portion of the cap or the outer circumference of the coupling member comprises an outer undercut which protrudes in relief, and a remaining one comprises an inner undercut which is formed in intaglio to correspond to the outer undercut.

5. The liquid container according to claim 1, further comprising:
a push part provided on a lower end of the cap and having an inward step to correspond to an upper end of the guide wall.

6. The liquid container according to claim 1 or 3, further comprising:
a locking step provided on an outer circumference of an upper portion of the inner stopper.

7. The liquid container according to claim 1 or 3, further comprising:
a sealing protrusion protruding from an upper surface of a central portion of the discharge plate of the inner stopper, and having a circumference which corresponds to an inner circumference of the passage of the opening control unit.

8. The liquid container according to claim 1 or 3, wherein a fastening protrusion protrudes from an outer surface of the outlet portion, and
a fastening groove is formed in an inner surface of the coupling inner wall of the outer stopper to correspond to the fastening protrusion.

9. The liquid container according to claim 1 or 3, wherein the passage of the opening control unit is formed such that an upper portion thereof is narrower than a lower portion thereof, thus preventing an excessive amount of contents from being discharged out of the main body.

10. The liquid container according to claim 1, further comprising:
an anti-removal protrusion provided on an outer surface of the upper portion of the opening control unit which is fitted into the through hole of the application member.

11. The liquid container according to claim 1 or 3, wherein the absorbent material of the application member is selected out of a group consisting of latex, sponge, and synthetic resin.
